# EUROPEAN PATENT APPLICATION

(11) **EP 4 215 166 A1**
(43) Date of publication of application: **26.07.2023**
(21) Application number: 23152716.9
(22) Date of filing: 20.01.2023
(51) Int. Cl.: A61F 2/966, A61B 17/11, A61F 2/06, A61F 2/04, A61F 2/07

(54) **A TRANSLUMINAL STENT DELIVERY AND POSITIONING SYSTEM**

(30) Priority: 21.01.2022 US 202217581663
(71) Applicant: Covidien LP, Mansfield, MA 02048 (US)
(72) Inventor: DESAI, Siddhi K., Mansfield, 02048 (US); MORRIS, Kevin M., Mansfield, 02048 (US); LEE, Kee Sein, Mansfield, 02048 (US); CARCAMO, Edward L., Mansfield, 02048 (US); LUBINSKI, Alexander A., Mansfield, 02048 (US)
(74) Representative: Maschio, Antonio

(57) **Abstract**

Methods, apparatuses, and systems are described for stent delivery and positioning for transluminal application. The method may include positioning the stent in an undeployed configuration through an access site in a wall of a first body lumen. In some cases, the method may include retracting an outer sheath proximally and past an anchoring component disposed at a distal portion of an inner tubular member based on positioning the stent. A distal portion of the stent may be disposed between the anchoring component and the outer sheath while the stent is in the undeployed configuration. The method may further include deploying the distal portion of the stent from the outer sheath and within the first body lumen and expanding a proximal portion of the stent from within the outer sheath such that upon fully exiting the outer sheath, the proximal portion expands to a deployed configuration within a second body lumen.

## Description

### BACKGROUND

Diseases and disorders of the gallbladder, pancreas, and bile ducts (i.e., pancreaticobiliary system) are associated with significant morbidity, mortality, and impaired quality of life. Obstructions, tumors, injuries, leakages, inflammation, infection, and lesions can occur in these structures, which can eventually lead to conditions such as biliary colic, cholecystitis, choledocholithiasis, cholelithiasis, pancreatitis, pancreatic duct stone formations, and chronic abdominal pain. Diseases of the pancreaticobiliary system may also be associated with nutritional disorders, such as malnutrition, obesity, and high cholesterol.

To treat a biliary obstruction, a clinician may perform a stent delivery procedure to place a stent across the body lumen to bypass the obstruction. In general, a stent delivery procedure may include placing an endoscope into the gastrointestinal tract and accessing the bile duct with a catheter. A guidewire may then be deployed through the catheter and into the bile duct. Once the guidewire is in place, a stent or other treatment device may be advanced over the guidewire into the bile duct. After the stent is placed in the bile duct, the clinician may withdraw the stent delivery system.

### SUMMARY

The described features generally relate to improved methods, systems, and devices for stent delivery and positioning for transluminal application. The method may include delivering the stent in an undeployed configuration through an access site of a body lumen and retracting an outer sheath proximally and past an anchoring component disposed at a distal portion of an inner tubular member. A distal portion of the stent may be disposed coaxially along the anchoring component such that the distal portion of the stent is disposed between the anchoring component and the outer sheath. In some cases, the distal portion of the stent may expand within the body lumen by retracting the outer sheath past the anchoring component. The proximal portion of the stent may expand from within the outer sheath such that the stent may then fully expand within a second body lumen.

A method for delivering a stent is described. The method may include positioning the stent in an undeployed configuration through an access site in a wall of a first body lumen, retracting an outer sheath proximally and past an anchoring component disposed at a distal portion of an inner tubular member based at least in part on positioning the stent, wherein a distal portion of the stent is disposed coaxially along the anchoring component such that the distal portion of the stent is disposed between the anchoring component and the outer sheath while the stent is in the undeployed configuration, deploying the distal portion of the stent from the outer sheath into a deployed configuration within the first body lumen based at least in part on retracting the outer sheath past the anchoring component, and expanding a proximal portion of the stent from within the outer sheath such that upon fully exiting the outer sheath, the proximal portion expands to a deployed configuration within a second body lumen.

The method may further include retaining the distal portion of the stent in place along the inner tubular member as the outer sheath is retracted past the anchoring component based at least in part on the distal portion of the stent being disposed between the anchoring component and the outer sheath. In some cases, deploying the distal portion of the stent may further include releasing the distal portion of the stent from the anchoring component and into the deployed configuration based at least in part on retracting the outer sheath past the anchoring component. In some cases, deploying the distal portion of the stent may further include expanding the distal portion of the stent from the undeployed configuration to the deployed configuration while the outer sheath is retracted based at least in part on a pressure being released between the anchoring component and the outer sheath as the outer sheath is retracted.

The method may further include compressing the distal portion of the stent in the undeployed configuration between the anchoring component and the outer sheath, wherein deploying the distal portion of the stent is based at least in part on compressing the distal portion of the stent. In some cases, deploying the distal portion of the stent may further include expanding a flared portion of the stent within the first body lumen based at least in part on retracting the outer sheath. The method may further include anchoring the flared portion of the stent within the first body lumen such that the distal portion of the stent remains in a fixed position.

The method may further include compressing the distal portion of the stent against the wall of the first body lumen based at least in part on deploying the distal portion of the stent from the outer sheath. The method may further include maintaining the anchoring component and the inner tubular member in a stationary position while retracting the outer sheath. The method may further include expanding an entire portion of the stent such that at least a portion of the stent extends through the first body lumen and into the second body lumen. The method may further include pulling the inner tubular member from a proximal end of the inner tubular member and removing the anchoring component and the inner tubular member from the first body lumen and the second body lumen after the stent fully deploys.

In some cases, the inner tubular member comprises a proximal marker around the inner tubular member and positioned such that a proximal end of the stent abuts against the proximal marker while the stent is in the undeployed configuration, and the method may further include aligning a distal end of the proximal marker with a wall of the second body lumen, maintaining the proximal marker in a stationary position while retracting the outer sheath based at least in part on aligning the distal end of the proximal marker, and withdrawing the outer sheath past the distal end of the proximal marker based at least in part on maintaining the proximal marker in the stationary position, wherein expanding the proximal portion of the stent is based at least in part on withdrawing the outer sheath past the proximal marker.

The method may further include withdrawing the outer sheath until the proximal portion of the stent fully exits the outer sheath based at least in part on deploying the distal portion of the stent. The method may further include advancing a stent delivery apparatus through the first body lumen and the second body lumen before delivering the stent and removing the stent delivery apparatus through the first body lumen and the second body lumen after expanding the stent. In some cases, the stent delivery apparatus comprises an electrocautery tip coupled with a distal end of the inner tubular member, and the method may include applying energy, via the electrocautery tip, to the wall of the first body lumen and accessing, via the access site, the first body lumen based at least in part on applying the energy to the wall of the first body lumen, wherein positioning the stent is based at least in part on accessing the first body lumen.

In some cases, the stent delivery apparatus comprises an isolation sheath, wherein the isolation sheath is disposed around the outer sheath, and wherein retracting the outer sheath may include maintaining the isolation sheath in a stationary position while retracting the outer sheath and withdrawing the outer sheath into the isolation sheath based at least in part on positioning the stent. In some cases, the stent delivery apparatus comprises a guidewire, wherein the guidewire is slidably disposed within the inner tubular member, and wherein the method further include advancing the guidewire through the access site before positioning the stent.

Certain embodiments of the present disclosure may include some, all, or none of the above advantages or features. One or more other technical advantages or features may be readily apparent to those skilled in the art from the figures, descriptions, and claims included herein. Moreover, while specific advantages or features have been enumerated above, various embodiments may include all, some, or none of the enumerated advantages or features.

Further scope of the applicability of the described methods and systems will become apparent from the following detailed description, claims, and drawings. The detailed description and specific examples are given by way of illustration only, since various changes and modifications within the spirit and scope of the description will become apparent to those skilled in the art.

### BRIEF DESCRIPTION OF THE DRAWINGS

In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1 illustrates a system for providing access to a body lumen in accordance with aspects of the present disclosure.
FIG. 2 illustrates a system for providing access to a body lumen in accordance with aspects of the present disclosure.
FIG. 3A illustrates a stent delivery system in accordance with aspects of the present disclosure.
FIG. 3B illustrates a stent delivery system within a body lumen in accordance with aspects of the present disclosure.
FIG. 3C illustrates a stent delivery system with a flared portion of the stent deployed in accordance with aspects of the present disclosure.
FIG. 3D illustrates a stent delivery system with the stent fully deployed in accordance with aspects of the present disclosure.
FIG. 3E illustrates a stent delivery system with the system removed in accordance with aspects of the present disclosure.
FIGs. 4 and 5 illustrate flowcharts of methods for stent delivery and positioning for transluminal application in accordance with aspects of the present disclosure.

### DETAILED DESCRIPTION

The present disclosure is generally directed to delivering a stent and positioning the stent for transluminal application. In certain procedures described herein, to place a stent within a body lumen, the luminal wall is pierced, and a stent delivery system is advanced through the hole (i.e., access site or access hole) and positioned at the target site to bypass an obstruction. The stent is then deployed from the stent delivery system, and the stent delivery system is withdrawn back out of the lumen through the same hole. If the stent is not accurately and precisely deployed, the stent may be unable to form a bridge between two body lumens and therefore, may be unable to bypass the obstruction. For example, if the distal portion of the stent is deployed short of (e.g., below) the access hole, the stent may be unable to connect the two body lumens and unable to form an alternate route to bypass the obstruction. In some cases, inaccurate deployment may result in fluid from the lumen leaking out into the surrounding tissue and organs, which may potentially cause serious discomfort or other medical complications.

In some cases, the stent may be positioned through an access site in a wall of a first body lumen after using a distal cutting element to create the access site. The stent may be in an undeployed configuration such that the stent is housed within the outer sheath. The system for delivering the stent may include an inner tubular member that is configured to advance through an access site in a wall of the body lumen. The stent may be disposed coaxially onto the inner tubular member. The outer sheath may be disposed coaxially along at least a portion of the inner tubular member such that the stent is disposed between the inner tubular member and the outer sheath while the stent is in an undeployed configuration.

The system for delivering the stent may further include an anchoring component disposed at a distal portion of the inner tubular member. In some cases, a distal portion of the stent may be disposed coaxially along the anchoring component such that the distal portion of the stent is disposed between the anchoring component and the outer sheath while the stent is in the undeployed configuration. The anchoring component may be configured to retain a distal portion of the stent in place along the inner tubular member as the outer sheath is retracted proximally to deploy the stent. For example, the outer sheath may be retracted proximally and past the anchoring component disposed at a distal portion of an inner tubular member while the anchoring component and the inner tubular memory remain stationary.

Upon retraction of the outer sheath, the stent releases from the anchoring component and expands into a deployed configuration within the body lumen. For example, the distal portion of the stent may deploy from the outer sheath into a deployed configuration within the first body lumen based on retracting the outer sheath past the anchoring component. In such cases, the distal portion of the stent may accurately and precisely deploy in the first body lumen, thereby enabling the stent to be able to form a bridge between two body lumens and bypass the obstruction. For example, the distal portion of the stent may deploy in the first body lumen and anchor itself within the first body lumen.

The stent may be an example of a non-foreshortening stent. For example, the stent may include a helical wrapping pattern that may be configured to reduce a foreshortening of the stent body upon deployment from the undeployed configuration to the deployed configuration to less than ten percent of a length of the stent body in the undeployed configuration. In such cases, the stent may remain in place during retraction of the outer sheath and deployment of the distal portion as the outer sheath continues to be retracted proximally. The non-foreshortening stent may enable accurate deployment by positioning the stent within the body lumen and maintaining the position of the stent within the body lumen before and after deploying the stent.

The system for delivering the stent may further include a proximal marker disposed around the inner tubular member and positioned such that a proximal end of the stent abuts against the proximal marker while the stent is in the undeployed configuration. The proximal marker may be configured to indicate a location of the proximal end of the stent within the system endoscopically and/or fluoroscopically. For example, a distal end of the marker may be aligned with a wall of the second body lumen to ensure that the proximal portion of the stent precisely deploys within the second body lumen and allows the stent to bridge between two body lumens upon expansion. The outer sheath may be withdrawn past the distal end of the marker to expand the proximal portion of the stent. In such cases, the proximal portion of the stent may expand from within the outer sheath such that upon fully exiting the outer sheath, the proximal portion expands to a deployed configuration within the second body lumen. The deployed configuration may be an example of the stent fully exiting the outer sheath and expanding between the first body lumen and the second body lumen, thereby providing an alternative route to bypass the obstruction.

In some cases, the stent may include a stent body having a first diameter and a first length in a deployed configuration. The stent may include a helical wrapping pattern that is at least partially covered with a material. In some cases, the stent may include at least two anchoring members coupled with a distal portion and a proximal portion, respectively, of the stent body. For example, the stent may include a first anchoring member coupled with a distal portion of the stent body and configured to increase a diameter of the distal portion of the stent body to a second diameter greater than the first diameter. The stent may further include a second anchoring member coupled with a proximal portion of the stent body and configured to increase a diameter of the proximal portion of the stent body to the second diameter greater than the first diameter. Each of the anchoring members may be configured to anchor the distal and proximal portions of the stent within the respective body lumens such that the stent remains in a fixed position.

Embodiments of the present disclosure are now described in detail with reference to the drawings. As used herein, the term "clinician" refers to a doctor, surgeon, nurse, or any other care provider and may include support personnel. The term "proximal" will refer to the portion of the device or component thereof that is closer to the clinician and the term 'distal" will refer to the portion of the device or component thereof that is farther from the clinician.

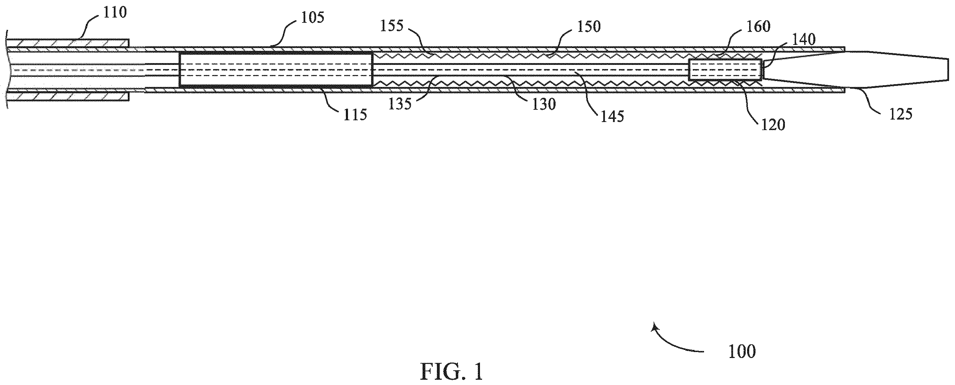

**FIG. 1** illustrates a system 100 for providing access to a body lumen and delivering a stent in accordance with aspects of the present disclosure. The system 100 generally includes an outer sheath 105, an isolation sheath 110, a proximal marker 115, an anchoring component 120, an electrocautery tip 125, an inner tubular member 130, a stent 150, and a guidewire 145. The system 100 can be provided as individual components, selectively combined components, or all together as a kit of components.

During a luminal access and stent 150 delivery procedure, the electrocautery tip 125 may access the target body lumen by piercing a wall of the body lumen, for example, to deliver a stent 150. In general, a stent 150 is a frame or scaffolding structure sized for placement within a body lumen and configured to provide structural support to the inner surface of the body lumen. A stent 150 may be used to restore patency across narrowed or blocked areas within the body lumen due to inflammation, tumors, plaque buildup, or any other obstructive feature. Although references to the pancreaticobiliary system are provided herein, it should be appreciated that the stents described herein may be used in any body lumen. Furthermore, as discussed in more detail below, the stent 150 may be disposed around the inner tubular member 130.

The stent 150 may be made from any number of materials, combinations of materials, and constructions. In some examples, the stent 150 is a self-expanding stent. The stent 150 may be a wire-form stent formed by one or more helically wrapped wires. However, it should be appreciated that the stent 150 may be made from other stent constructions or combinations of stent constructions. In other examples, the stent 150 is a laser-cut stent formed from a single metallic tube with regions cut away for increased flexibility. In yet other examples, the stent 150 is a braided stent made from a plurality of wires joined together in a cross-hatch configuration. In some examples, the stent 150 may be a combination of the braided stent and the wire-form stent.

It may be appreciated that the different stent constructions may exhibit particular characteristics such as radial expansive force, flexibility, reduced foreshortening, or migration resistance that may render a certain construction advantageous for a particular use. For example, the helical wrapping pattern of the stent 150 may be configured to reduce a foreshortening of the stent body upon deployment from an undeployed configuration to the deployed configuration to less than ten percent of a length of the stent body in the undeployed configuration. In such cases, the stent 150 may be an example of a non-foreshortening stent.

The individual wires or frame of the stent 150 may be made from any number of metallic materials including, but not limited to, titanium, nitinol, or stainless steel. It should be appreciated that other metallic or non-metallic materials may be used to construct the stent 150 that provides suitable flexibility, stiffness, and biocompatibility. The stent 150 may include a polymeric or fabric sleeve (e.g., first material) that covers some or all of the surface of the stent 150. Such a sleeve may protect the inner surface of the body lumen from the bare metal of the stent 150 and may prevent tissue ingrowth. For example, the stent 150 may include a helical wrapping pattern that is at least partially covered with a first material. In some examples, the stent 150 is a drug-eluting stent.

The outer sheath 105 of the system 100 has an elongate tubular body and an internal lumen extending from its proximal end to the distal end. In general, the outer sheath 105 may be configured to access a body lumen and to provide a conduit through which one or more devices (e.g., a guidewire 145) may pass to facilitate subsequent treatment of the body lumen or associate organs. The outer sheath 105 may include features that facilitate the direction-controlled delivery of a guidewire 145 within the body lumen for subsequent delivery of a stent 150, a biopsy device, a medicinal delivery element, or any number of other treatment or diagnostic devices.

The outer sheath 105 may be disposed coaxially along at least a portion of the inner tubular member 130 such that the stent 150 is disposed between the inner tubular member 130 and the outer sheath 105 while the stent 150 is in an undeployed configuration. The undeployed configuration may be an example of a stent 150 constrained within the outer sheath 105, an unexpanded configuration of the stent 150, or both. In some cases, the outer sheath 105 may include a braided extrusion in which a braided, distal section of the outer sheath 105 is fused with a braided, proximal section of the outer sheath 105. In some cases, the outer sheath 105 may include a constant diameter along the length of the outer sheath 105.

In some examples, the outer sheath 105 may include a lubrication coating disposed within an inner surface of the outer sheath 105. The lubrication coating may be made from a variety of materials, including but not limited to silicone. In such cases, the lubrication coating may reduce deployment forces by at least thirty percent to ensure accurate placement of the stent 150 within the body lumen. In some cases, the lubrication coating of the outer sheath 105 may reduce the friction between the outer sheath 105 and the stent 150 as the outer sheath 105 is retracted over the stent 150. The isolation sheath 110 may be configured to receive the outer sheath 105 as the outer sheath 105 is retracted. The isolation sheath 110 may include a lubrication coating disposed within an inner surface of the isolation sheath 110.

The inner tubular member 130 is generally an elongate, tubular member with a proximal end 135 and distal end 140 and is dimensioned to be advanced through the internal lumen of the outer sheath 105. The inner tubular member 130 may be configured to advance through an access site in a wall of the body lumen. In certain embodiments, the inner tubular member 130 includes one or more internal lumens extending from the proximal end 135 to the distal end 140 to house a power wire coupled with the electrocautery tip 125 in one internal lumen and the guidewire 145 in another internal lumen. The inner tubular member 130 may extend from a proximal end of the electrocautery tip 125 to a lure at the handle end and provide a passageway for the guidewire 145. As described below, the inner tubular member 130 is configured to house the guidewire 145. The inner tubular member 130 may be made of a number of materials, but not limited to polyether ether ketone (PEEK), polytetrafluoroethylene (PTFE), polyimide, or both.

The inner tubular member 130 may be coupled with an anchoring component 120 at the distal end 140 of the inner tubular member 130. In certain embodiments, the distal end 140 of the inner tubular member 130 includes a tip or bulged portion (e.g., anchoring component 120). The stent 150 may be coupled to the inner tubular member 130 and the anchoring component 120. For example, the stent 150 may be concentric with the inner tubular member 130 and the anchoring component 120. As such, the inner tubular member 130 may extend through the lumen of the stent 150. For example, the stent 150 may be disposed coaxially onto the inner tubular member 130. The stent 150 may be positioned between the outer sheath 105 and the inner tubular member 130 at the proximal end 135 of the inner tubular member 130.

The anchoring component 120 may extend through the lumen of the stent 150 at a distal portion 160 of the stent 150. In such cases, the stent 150 may be positioned between the outer sheath 105 and the anchoring component 120 at the distal end 140 of the inner tubular member 130. For example, the distal portion 160 of the stent 150 may be disposed coaxially along the anchoring component 120 such that the distal portion 160 of the stent 150 is disposed between the anchoring component 120 and the outer sheath 105 while the stent 150 is in the undeployed configuration.

The anchoring component 120 may be made from a variety of materials, including but not limited to silicone. The anchoring component 120 may be disposed at a distal end 140 of the inner tubular member 130 and configured to retain a distal portion 160 of the stent 150 in place along the inner tubular member 130 as the outer sheath 105 is retracted proximally to deploy the stent 150. As described below in further detail, upon retraction of the outer sheath 105, the stent 150 may release from the anchoring component 120 and expand into a deployed configuration within the body lumen. The deployed configuration may be an example of an unconstrained configuration, an expanded configuration, or both.

The anchoring component 120 may be an example of a bump, an increased diameter component of the inner tubular member 130, a hook, or a combination thereof. In such cases, the anchoring component 120 may be configured to keep the distal portion 160 of the stent 150 stationary as the outer sheath 105 is retracted. In some examples, the anchoring component 120 may be 8 mm in length or 4 cm in length for a stent 150 that is 8-10 cm in length. In some cases, the inner tubular member 130 may include a single anchoring component 120 or more than one anchoring component 120. For example, the inner tubular member 130 may include at least three anchoring components 120 made of polyether block amide (PEBA) and positioned along the inner tubular member 130. The anchoring components 120 may extend 1 cm higher from the outer surface of the inner tubular member 130.

The system 100 may further include a proximal marker 115. The proximal marker 115 may be an example of a proximal marker that is disposed around the inner tubular member 130 and positioned such that a proximal portion 155 of the stent 150 abuts against the proximal marker 115 while the stent 150 is in the undeployed configuration. The proximal marker 115 may be configured to indicate a location of the proximal portion 155 of the stent 150 within the system 100. The proximal marker 115 may be configured to retain a proximal portion 155 of the stent 150 in place along the inner tubular member 130 as the outer sheath 105 is retracted proximally to deploy the stent 150. In some cases, the proximal marker 115 may be coupled with the inner tubular member 130 such that the proximal marker 115 remains stationary as the outer sheath 105 is retracted.

The proximal marker 115 includes generally an elongate, tubular member and is configured to house the inner tubular member 130. In some cases, the proximal marker 115 may be tapered such that a distal end of the proximal marker 115 may extend underneath the proximal portion 155 of the stent 150. For example, the proximal marker 115 may be an example of a proximal anchoring component such that the proximal marker 115 may be configured to compress the proximal portion 155 of the stent 150 between the proximal marker 115 and the outer sheath 105.

The electrocautery tip 125 may be an example of a distal cutting element coupled with the distal end 140 of the inner tubular member 130 and configured to create the access site in the wall of the body lumen. The electrocautery tip 125 may include a coiled electrode wire that extends radially around a circumference of a distal end of the electrocautery tip 125, a single electrode wire that extends longitudinally and in a proximal direction from a distal end of the electrocautery tip 125, a single, spiral electrode wire that extends around a distal end of the electrocautery tip 125, or an electrode tube. In some case, the electrocautery tip 125 may include a tapered cover disposed around the electrocautery tip 125. The outer sheath 105 may at least partially overlap the tapered cover. The tapered cover may be made from a variety of materials, including but not limited to silicone or other flexible materials. In some cases, the outer diameter of the electrocautery tip 125 may be equal to the inner diameter of the outer sheath 105. In other examples, the outer diameter of the electrocautery tip 125 may be greater than the inner diameter of the outer sheath 105.

The guidewire 145 is generally a flexible elongate member configured to slidably advance through the internal lumen of the inner tubular member 130. In such cases, the guidewire 145 may be disposed within the inner tubular member 130. The guidewire 145 may be uniform in size and stiffness along its entire length, or alternatively, may include sections of differing stiffness.

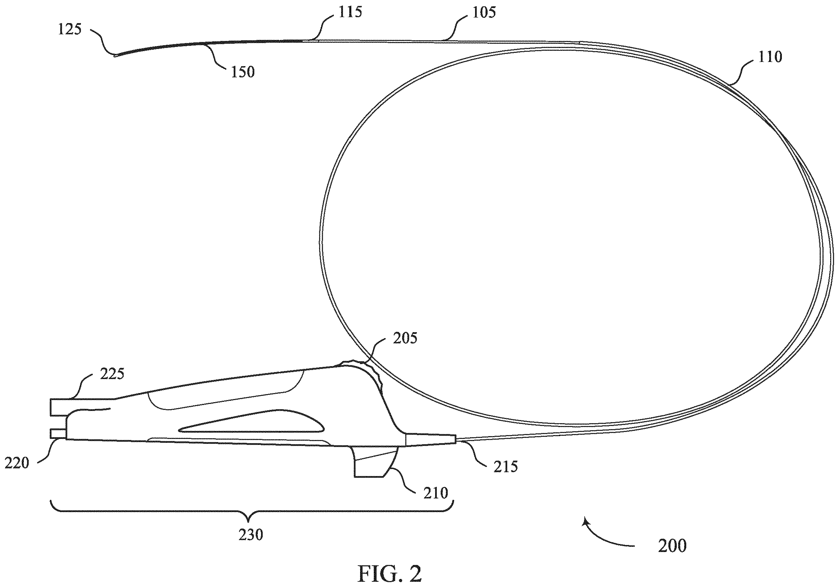

**FIG. 2** illustrates a system 200 for providing access to a body lumen in accordance with aspects of the present disclosure. The system 200 generally includes the outer sheath 105, the isolation sheath 110, the proximal marker 115, the anchoring component 120 (not shown), the electrocautery tip 125, the inner tubular member 130 (not shown), and the stent 150, which may be examples of the corresponding components described with reference to FIG. 1. The system 200 may also include a thumbwheel 205, a stationary member 210, a guidewire lumen 220, and a connector port 225. The system 200 can be provided as individual components, selectively combined components, or all together as a kit of components.

The thumbwheel 205 may be coupled with a proximal end 215 of the isolation sheath 110 and configured to retract the outer sheath 105 proximally. In some cases, the outer sheath 105 may be bonded with a plastic component at a proximal end of the outer sheath 105 to the handle assembly 230. The actuation of the thumbwheel 205 may retract the outer sheath 105 to deploy the stent 150. As described below, the outer sheath 105 may be retracted into the stationary isolation sheath 110.

The stationary member 210 may be an example of a locking pin. The stationary member 210 may be configured to prevent deployment when the device is inserted into the scope. For example, features of the stationary member 210 may engage into the carriage to prevent accidental deployment. To begin actuation of the thumbwheel 205, the stationary member 210 may be removed from the handle assembly 230. In such cases, the device may be deployed based on removing the stationary member 210 and actuating the thumbwheel 205.

In some cases, the isolation sheath 110 may stabilize the device against the scope channel and isolate friction that the catheter may experience otherwise in tortuosity. In such cases, the system 200 may enable accurate deployment of the stent 150 through the sheath-based delivery system 200 (e.g., including at least the isolation sheath 110 and the outer sheath 105) to ensure stability of the catheter against the scope. In some cases, the isolation sheath 110 may include two different diameters along the length of the isolation sheath 110 to customize interaction with the scope channel.

The isolation sheath 110 may made of a material such as high-density polyethylene (HDPE). The isolation sheath 110 may be in contact with the inner diameter of the working channel of the endoscope, thereby inducing friction between the isolation sheath 110 and working channel. As the thumbwheel is actuated the outer sheath 105 is retracted, the friction between the isolation sheath 110 and the working channel may be greater than the friction between the isolation sheath 110 and the outer sheath 105 such that the isolation sheath 110 remains stationary as the outer sheath 105 is retracted. In such cases, the isolation sheath 110 may be configured as a barrier between the outer sheath 105 and the working channel, thereby reducing the overall friction on the system 200.

The outer sheath 105 may be inserted into a handle assembly 230, and once assembled, the outer sheath 105 extends through the handle assembly 230 to the target body lumen. In some cases, a power wire may be connected to the electrocautery tip 125 and may be laminated on the inner lumen member by a polyester heat shrink. In such cases, the power wire may extend through the handle assembly 230 and through the connector port 225. The electrode of the electrocautery tip 125 may be connected through the connector port 225 in the handle assembly 230. In some cases, the electrode may connect to a radiofrequency (RF) generator.

The guidewire lumen 220 may generally be a tubular structure that is sized to deploy the stent 150 within the body lumen. The guidewire lumen 220 may access the human body through the working channel of an endoscope, for example. As will be appreciated, the guidewire lumen 220 may be made from any number of biocompatible materials or combinations of materials suitable for medical sheaths, catheters, and the like.

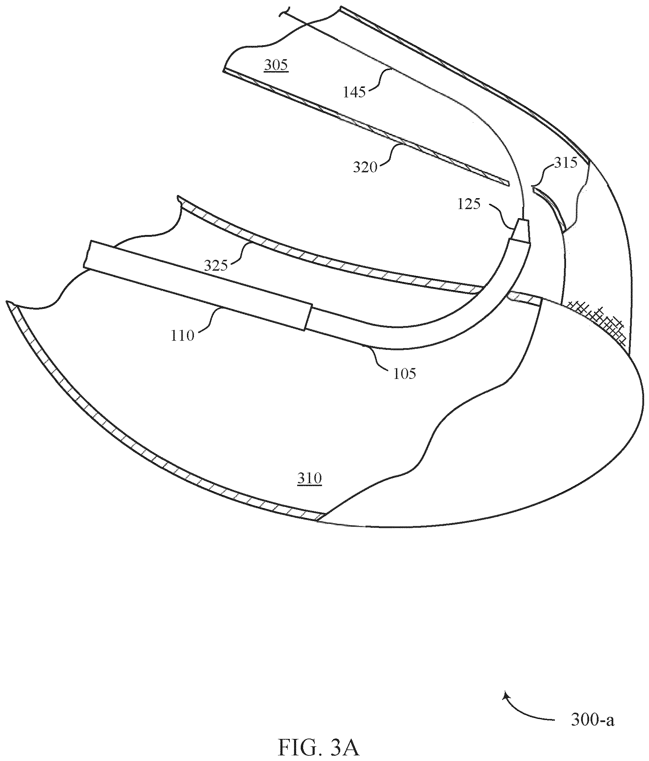

**FIG.** 3A illustrates a stent delivery system 300-a in accordance with aspects of the present disclosure. The stent delivery system 300-a may be configured to place a stent (not shown) within a first body lumen 305 to restore luminal flow from a first body lumen 305 to a second body lumen 310, thereby bypassing narrowed areas or blockages within at least the first body lumen 305. The stent delivery system 300-a may be sized or otherwise adapted to place a stent within any body lumen, such as those associated with the pancreaticobiliary system, the arterial system, the bronchial system, the urinary system, or any other luminal system that may require stent treatment.

The stent delivery system 300-a may generally include the isolation sheath 110, the outer sheath 105, the electrocautery tip 125, and the guidewire 145, which may be examples of the corresponding components described with reference to FIGs. 1 and 2. The guidewire 145 may be part of the stent delivery system 300-a or may be a separate component. The stent delivery system 300-a can be provided as individual components, selectively combined components, or all together as a kit of components.

With reference to FIG. 3A, a stent delivery system 300-a for providing access to a body lumen is illustrated in accordance with aspects of the present disclosure. The stent delivery system 300-a may be examples of or include functionality of the systems or components described with reference to any of FIGS. 1 and 2. The illustrated portions of the system include the first body lumen 305 (e.g., a common bile duct), which drains bile from both the cystic duct (which drains from the gallbladder) and the common hepatic duct (which drains from the liver) into the second body lumen 310 (e.g., duodenum), where the bile mixes and reacts with digesting food.

A clinician may advance an endoscope (e.g., an EUS endoscope) into the lumen of a patient's duodenum (e.g., second body lumen 310) to a position in which the bile ducts may be visualized (e.g., via endosonography). The clinician may then access the common bile duct (e.g., first body lumen 305) by advancing a separate access device from a working channel of the endoscope, through the wall 325 of the duodenum (i.e., trans-duodenally), and then through the wall 320 of the common bile duct.

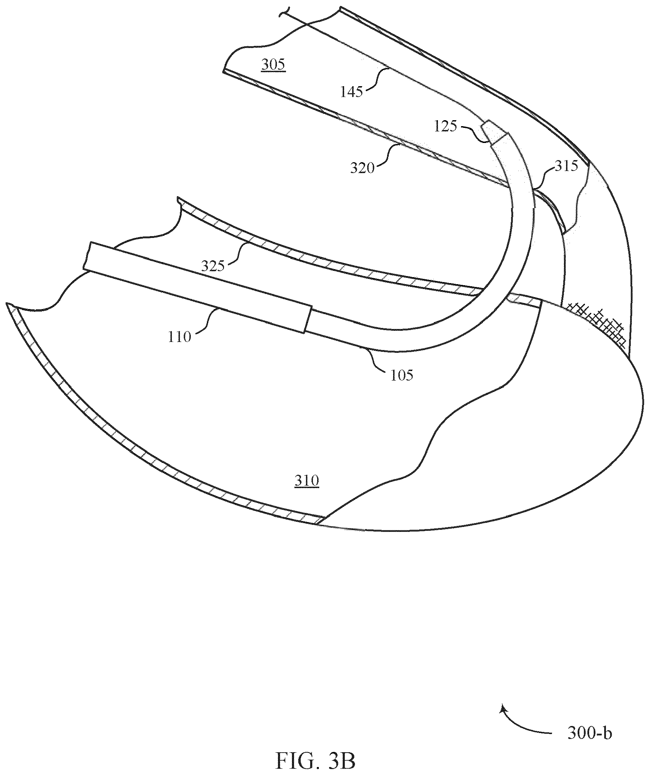

The clinician may then insert a guidewire 145 via the separate access device, thereby allowing the stent delivery system 300-a to be tracked over the guidewire 145. After the stent delivery system 300-a is advanced into the common bile duct, the stent delivery system 300-a may retract the outer sheath 105 to position the stent through the first body lumen 305 and the second body lumen 310, as described in reference to FIGs. 3B-3E.

During a luminal access and stent delivery procedure, the electrocautery tip 125 may access the target lumen (e.g., first body lumen 305) by piercing a wall 320 of the first body lumen 305, for example. In some examples, a sharpened stylet may be used in conjunction with the electrocautery tip 125 to facilitate piercing the wall 320 (e.g., luminal wall). For example, the sharpened stylet may be advanced through the electrocautery tip 125 until it protrudes from the electrocautery tip 125 to pierce tissue. In some cases, the guidewire 145 may be advanced through the outer sheath 105 and into the first body lumen 305 as the electrocautery tip 125 accesses the first body lumen 305. The electrocautery tip 125 may access the target lumen (e.g., first body lumen 305) by piercing a wall 325 of the second body lumen 310 prior to piercing the wall 320 of the first body lumen 305. In such cases, the electrocautery tip 125 may exit the second body lumen 310 and target access of the first body lumen 305. The first body lumen 305 may be an example of the biliary duct, and the second body lumen 310 may be an example of the duodenum.

Referring still to FIG. 3A, to place the stent delivery system 300-a within the first body lumen 305, an access site 315 is formed through the wall 320 of the first body lumen 305, and the guidewire 145 is then advanced through the access site 315 and into the first body lumen 305. In some cases, to access the first body lumen 305, the electrocautery tip 125 may apply energy to the wall 320 of the first body lumen 305 and access, via the access site 315, the first body lumen 305 based on applying the energy to the wall 320 of the first body lumen 305. In some cases, prior to accessing the first body lumen 305, the electrocautery tip 125 may apply energy to the wall 325 of the second body lumen 310 and access the first body lumen 305 based on applying the energy to the wall 325 of the second body lumen 310. For example, the electrocautery tip 125 may cut the tissue in contact with the electrode of the electrocautery tip 125. The stent delivery system 300-a (e.g., a stent delivery apparatus) may be advanced through the first body lumen 305 and the second body lumen 310.

The stent delivery system 300-a may be used to access and provide treatment to one or more body lumens within the gastrointestinal system or pancreaticobiliary system, for example. It may be appreciated that the stent delivery system 300-a may also be used to provide access or treatment to other organs or luminal systems within the body such as the arterial system, the bronchial system, the urinary system, or any other luminal system were maneuverability and accuracy is desirable.

The stent delivery system 300-a may be configured for choledochoduodenostomy (CDS) and hepaticogastrostomy (HGS) procedures in which the stent is implanted across two tissues layers (e.g., duodenum to common bile duct or stomach to intrahepatic duct). After the biliary duct is accessed and guidewire 145 is placed, the tract across the two tissues is dilated mechanically or via electrocautery followed by insertion of stent delivery system 300-a to implant the stent. The tract dilation step may increase a rate of acute adverse events. In some cases, accurate stent deployment may be dependent on operator skillset and the operator may dynamically adjust the delivery system position after deployment is initiated and the stent is deployed. Therefore, techniques may be desired to utilize the stent delivery system 300-a to enable increased deployment accuracy within the body lumen.

In some cases, the stent delivery system 300-a may be configured for transmittal biliary drainage. In such cases, the stent may bridge between the second body lumen 310 (e.g., the duodenum) and a portion of the first body lumen 305 (e.g., the biliary duct) to create a bridge to bypass an obstruction. The obstruction may be an example of a distal malignant biliary obstruction that obstructs drainage. To access the first body lumen 305, the stent delivery system 300-a may perform a track dilation based on a mechanical dilation via a balloon or electrical dilation via a knife. In some cases, the track dilation may be performed by the electrocautery tip 125. In such cases, the stent delivery system 300-a may enable the track dilation step to be performed together with the delivery the stent as the electrocautery tip 125 is coupled with the distal end of the inner tubular member within the outer sheath 105.

In some examples described herein, the handle assembly is coupled with an endoscope and the outer sheath 105 is guided via endoscopic ultrasound (EUS) to provide access to one or more body lumens or organs associated with the pancreaticobiliary system for the purpose of providing treatment. For example, the stent delivery system 300-a may be configured to provide access to at least the common biliary duct to facilitate subsequent procedures to treat narrowed areas or blockages within the bile duct and create a bypass around the narrowed areas or blockages within the bile duct to provide access to the stomach and from the biliary duct via the stent.

**FIG. 3B** illustrates a stent delivery system 300-b within the first body lumen 305 in accordance with aspects of the present disclosure. Once the guidewire 145 is in place, the outer sheath 105 is advanced into the first body lumen 305, over the guidewire 145, through the access site 315, and into the first body lumen 305.

The stent (not shown) may be positioned, in the undeployed configuration, through an access site 315 in a wall 320 of a first body lumen 305. In such cases, the stent may be constrained within the outer sheath 105, and the outer sheath 105 may be advanced through the access site 315. The stent may be positioned in the first body lumen 305 after advancing the stent delivery apparatus through the first body lumen 305 and the second body lumen 310. In such cases, the stent may be positioned based on accessing the first body lumen 305, accessing the second body lumen 310, or both. In some cases, the stent may be positioned after advancing the guidewire 145 through the access site 315.

The inner tubular member (not shown) may be operatively coupled with the stent. As such, the inner tubular member and stent may be advanced over the guidewire 145 and into the first body lumen 305. As discussed in more detail below, the stent may be attached to the inner tubular member between the outer sheath 105 and the anchoring component (not shown) coupled with the inner tubular member.

Once in the first body lumen 305, the outer sheath 105, the inner lumen member, and the anchoring component may be advanced distally. In some cases, the outer sheath 105 and the inner lumen member may be advanced distally such that the outer sheath 105 and the inner lumen member extend through and into the biliary duct. The outer sheath 105 and the inner lumen member may be advanced until a distal end of the marker (not shown) is aligned with the wall 325 of the second body lumen 310. In such cases, prior to retracting the outer sheath 105, the anchoring component may be positioned within the first body lumen 305, and the marker may be positioned within the second body lumen 310 such that the stent traverses the first body lumen 305 and the second body lumen 310.

The outer sheath 105 may be disposed around the inner lumen member and the anchoring component. As such, the distal portion of the stent is disposed between the anchoring component and the outer sheath 105, and the proximal portion of the stent is disposed between the inner lumen member and the outer sheath 105. For example, the distal portion of the stent may be compressed in the undeployed configuration between the anchoring component and the outer sheath. In some cases, the access site 315 may be covered by the outer sheath 105.

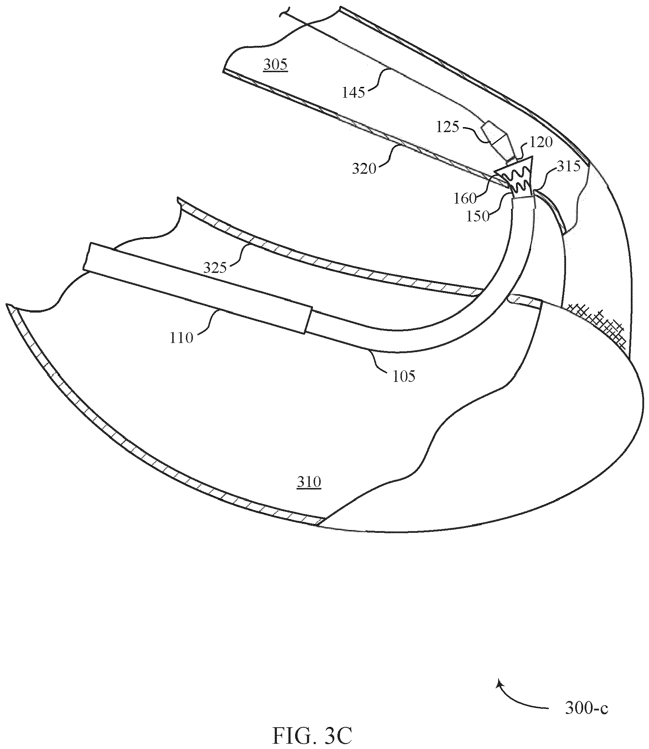

**FIG. 3C** illustrates a stent delivery system 300-c with a flared portion of the stent 150 deployed in accordance with aspects of the present disclosure. Once the outer sheath 105 is removed through the access site 315, the distal portion 160 of the stent 150 may deploy. As the outer sheath 105 is withdrawn through the access site 315, the inner lumen member (not shown) and the anchoring component 120 may remain stationary, and the distal portion 160 of the stent 150 may be exposed within the first body lumen 305. Once the desired anatomical position of the stent 150 has been achieved within the first body lumen 305, the outer sheath 105 may be retracted.

The outer sheath 105 may be retracted proximally and past the anchoring component 120 disposed at a distal portion of an inner tubular member based on positioning the stent 150. For example, the distal portion 160 of the stent 150 may be disposed coaxially along the anchoring component 120 such that the distal portion 160 of the stent 150 is disposed between the anchoring component 120 and the outer sheath 105 while the stent 150 is in the undeployed configuration. The distal portion 160 of the stent 150 may be deployed from the outer sheath 105 into a deployed configuration within the first body lumen 305 based on retracting the outer sheath 105 past the anchoring component 120.

In some cases, the distal portion 160 of the stent 150 may be released from the anchoring component 120 and into the deployed configuration in response to retracting the outer sheath 105 past the anchoring component 120. For example, the distal portion 160 of the stent 150 may expand from the undeployed configuration to the deployed configuration while the outer sheath 105 is retracted based on a pressure being released between the anchoring component 120 and the outer sheath 105 as the outer sheath 105 is retracted. In such cases, the distal portion 160 of the stent 150 is no longer compressed between the outer sheath 105 and the anchoring component 120 and is free to expand within the first body lumen 305.

The distal portion 160 of the stent 150 may include a flared portion. In such cases, the flared portion may expand within the first body lumen 305 in direct response to retracting the outer sheath 105 past the anchoring component 120. As the outer sheath 105 is removed through the access site 315, the distal portion 160 of the stent 150 expands to expose the flared portion. As the distal portion 160 of the stent 150 expands, the flared portion contacts the wall 320 of the first body lumen 305. The flared portion (e.g., distal portion 160) of the stent 150 may be anchored within the first body lumen 305 such that the distal portion 160 of the stent 150 remains in a fixed position. In that case, the distal portion 160 prevents the stent 150 from being further withdrawn through the access site 315. The clinician may be able to feel the resistance of the flared portion against the first body lumen 305 and may therefore infer the location of the stent 150. Additionally or alternatively, the distal portion 160 of the stent 150 may be viewed under fluoroscopy or similar imaging techniques to infer the location of the stent 150.

The distal portion 160 of the stent 150 may be retained in place along the inner tubular member as the outer sheath 105 is retracted past the anchoring component 120 based on the distal portion 160 of the stent 150 being disposed (e.g., compressed) between the anchoring component 120 and the outer sheath 105. While the outer sheath 105 is retracted, the anchoring component 120 and the inner tubular member may be maintained in a locked position (e.g., stationary with respect to the withdrawn outer sheath 105). As the outer sheath 105 is retracted, the isolation sheath 110 is maintained in a locked position while retracting and withdrawing the outer sheath 105 into the isolation sheath 110.

The friction between the anchoring component 120 and the distal portion 160 of the stent 150 may keep the stent 150 in place along the inner tubular member as the outer sheath 105 is retracted. In some cases, the friction between the stent 150 and the outer sheath 105 may keep the stent 150 in place as the outer sheath 105 is retracted such that after the outer sheath 105 is retracted past the anchoring component 120 (e.g., clears the anchoring component 120), the distal portion 160 of the stent 150 expands from an undeployed to a deployed configuration.

In such cases, the outer sheath 105 may be retracted to a first position (e.g., past a distal end of the anchoring component 120), and the distal portion 160 of the stent 150 may deploy in a same location as compared to a location prior to retracting the outer sheath 105. Once the outer sheath 105 is retracted past the distal end of the anchoring component 120, the distal portion 160 of the stent 150 that was positioned between the outer sheath 105 and the anchoring component 120 may expand into the first body lumen 305 and anchor itself to the first body lumen 305. In such cases, the anchored distal portion 160 of the stent 150 may maintain the stent in a stationary position as the outer sheath 105 is retracted.

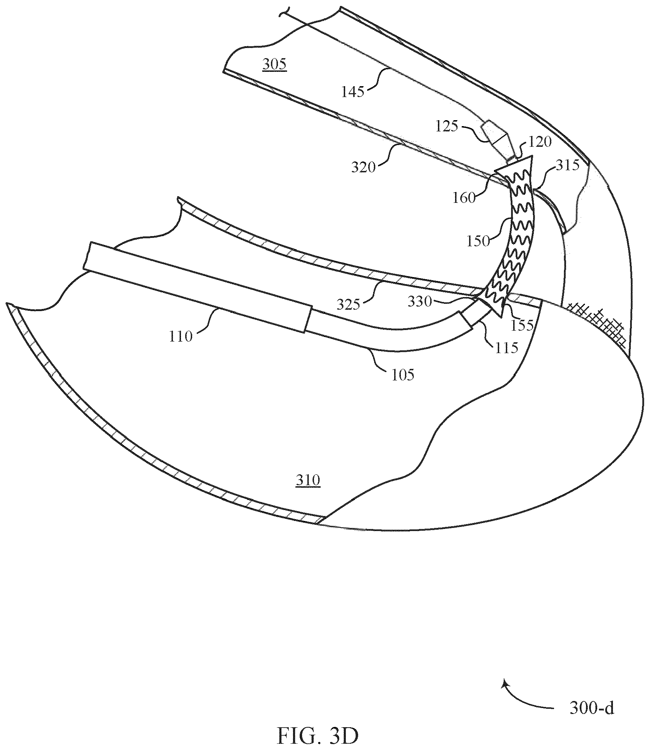

**FIG. 3D** illustrates a stent delivery system 300-d with the stent 150 fully deployed in accordance with aspects of the present disclosure. As the outer sheath 105 is further withdrawn proximally, the inner lumen member (not shown) may remain stationary, and the stent 150 may be exposed within the first body lumen 305 and into the second body lumen 310. To deploy the stent 150 within the first body lumen 305 and second body lumen 310, the outer sheath 105 may be retracted past a distal end 330 of the proximal marker 115. In the case of a non-foreshortening stent, the stent 150 expands to contact the inner surface of the first body lumen 305 and the inner surface of the second body lumen 310 such that the stent 150 forms a bridge between the first body lumen 305 and the second body lumen 310.

The distal end 330 the proximal marker 115 may be aligned with the wall 325 of the second body lumen 310. While the outer sheath 105 is retracted, the proximal marker 115 may be maintained in a locked position after aligning the distal end 330 of the proximal marker 115 with the wall 325 of the second body lumen 310. Once the outer sheath 105 is retracted past the distal end 330 of the proximal marker 115, the proximal portion 155 of the stent 150 may expand from within the outer sheath 105 in response to withdrawing the outer sheath 105 past the proximal marker 115. For example, the proximal portion 155 of the stent 150 may expand from within the outer sheath 105 such that upon fully exiting the outer sheath 105, the proximal portion 155 expands to a deployed configuration within the second body lumen 310. In such cases, the entire portion of the stent 150 may expand such that at least a portion of the stent 150 extends through the first body lumen 305 and into the second body lumen 310.

The proximal portion 155 of the stent 150 may be retained in place along the inner tubular member as the outer sheath 105 is retracted past the proximal marker 115 based on the proximal portion 155 of the stent 150 being disposed (e.g., compressed) between the inner lumen member and the outer sheath 105. In some cases, the proximal portion 155 of the stent 150 may be retained in place as the outer sheath 105 is retracted based on the proximal portion of the stent 150 abutting a proximal end of the proximal marker 115. While the outer sheath 105 is retracted, the inner tubular member may be maintained in a locked position (e.g., stationary with respect to the withdrawn outer sheath 105). As the outer sheath 105 is retracted, the isolation sheath 110 is maintained in a locked position while retracting and withdrawing the outer sheath 105 into the isolation sheath 110.

In such cases, the outer sheath 105 may be retracted to a second position (e.g., past the distal end 330 of the proximal marker 115), and the proximal portion 155 of the stent 150 may deploy in a same location as compared to a location prior to retracting the outer sheath 105. Once the outer sheath 105 is retracted past the distal end 330 of the proximal marker 115, the proximal portion 155 of the stent 150 that was positioned between the outer sheath 105 and the inner lumen member may expand into the second body lumen 310 and anchor itself to the second body lumen 310. In such cases, the anchored proximal portion 155 of the stent 150 may maintain the stent 150 in a stationary position as the inner lumen is retracted after the stent 150 fully deploys.

The proximal portion 155 of the stent 150 may include a flared portion. In such cases, the flared portion may expand within the second body lumen 310 in direct response to retracting the outer sheath 105 past the distal end 330 of the proximal marker 115. As the outer sheath 105 is withdrawn past the proximal marker 115 and into the isolation sheath 110, the proximal portion 155 of the stent 150 expands to expose the flared portion. As the proximal portion 155 of the stent 150 expands, the flared portion contacts the wall 325 of the second body lumen 310. The flared portion (e.g., proximal portion 155) of the stent 150 may be anchored within the second body lumen 310 such that the proximal portion 155 of the stent 150 remains in a fixed position.

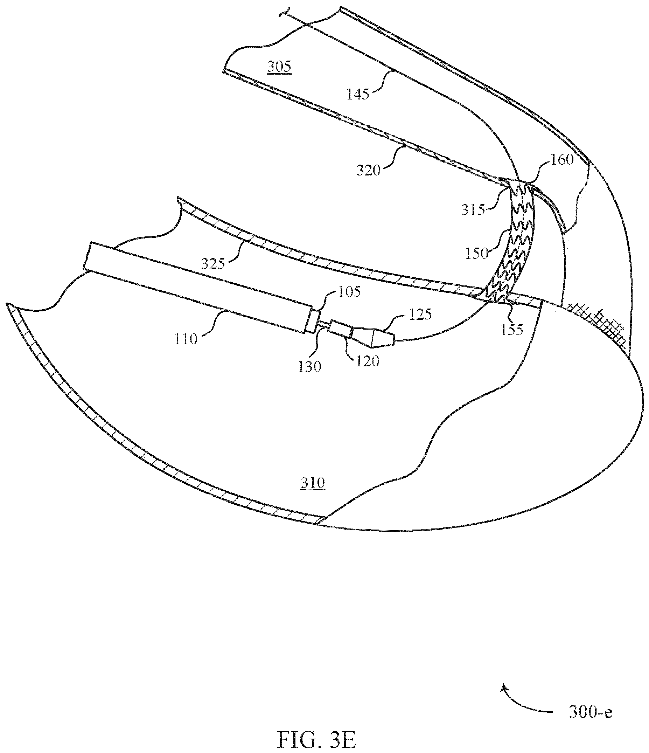

**FIG. 3E** illustrates a stent delivery system 300-e with the system removed in accordance with aspects of the present disclosure. Once the stent 150 fully expands, the inner tubular member 130, the anchoring component 120, the electrocautery tip 125, and the guidewire 145 are withdrawn through the access site 315. Once the outer sheath 105 is fully retracted into the isolation sheath 110, the inner tubular member 130, the anchoring component 120, and the electrocautery tip 125 may be removed from the first body lumen 305, through the access site 315, and from the second body lumen 310 after the stent 150 fully deploys. In such cases, the inner tubular member 130 and the anchoring component 120 may be retracted into the outer sheath 105 until the distal end of the outer sheath 105 at least partially overlaps with the electrocautery tip 125. For example, the inner tubular member 130 may be pulled from a proximal end of the inner tubular member 130. The stent delivery apparatus (e.g., stent delivery system 300-e) may be removed through the first body lumen 305 and the second body lumen 310 after expanding the stent 150.

The flared portion (e.g., distal portion 160) of the stent 150 may be anchored within the first body lumen 305 such that the distal portion 160 of the stent 150 remains in a fixed position. In such cases, the distal portion 160 of the stent 150 may be compressed against the wall 320 of the first body lumen 305 based on deploying the distal portion 160 of the stent 150 from the outer sheath 105. Furthermore, the stent 150 may at least partially cover the access site 315.

The flared portion (e.g., proximal portion 155) of the stent 150 may be anchored within the second body lumen 310 such that the proximal portion 155 of the stent 150 remains in a fixed position. In such cases, the proximal portion 155 of the stent 150 may be compressed against the wall 325 of the second body lumen 310 based on expanding the proximal portion 155 of the stent 150 from within the outer sheath 105. Furthermore, the stent 150 may at least partially cover the access site of the second body lumen 310.

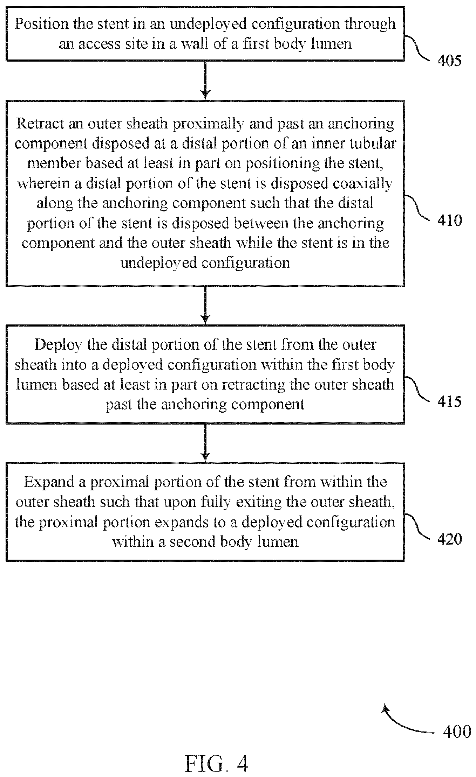

**FIG.** 4 illustrates a flowchart of a method 400 for stent delivery and positioning for transluminal application in accordance with aspects of the present disclosure.

At 405, the method may include positioning the stent in an undeployed configuration through an access site in a wall of a first body lumen. The operations of 405 may be performed in accordance with examples as disclosed herein.

At 410, the method may include retracting an outer sheath proximally and past an anchoring component disposed at a distal portion of an inner tubular member based at least in part on positioning the stent, wherein a distal portion of the stent is disposed coaxially along the anchoring component such that the distal portion of the stent is disposed between the anchoring component and the outer sheath while the stent is in the undeployed configuration. The operations of 410 may be performed in accordance with examples as disclosed herein.

At 415, the method may include deploying the distal portion of the stent from the outer sheath into a deployed configuration within the first body lumen based at least in part on retracting the outer sheath past the anchoring component. The operations of 415 may be performed in accordance with examples as disclosed herein.

At 420, the method may include expanding a proximal portion of the stent from within the outer sheath such that upon fully exiting the outer sheath, the proximal portion expands to a deployed configuration within a second body lumen. The operations of 420 may be performed in accordance with examples as disclosed herein.

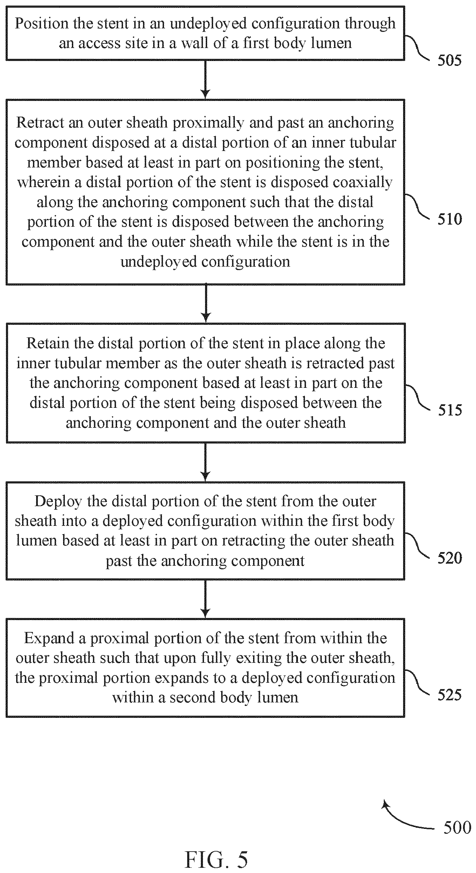

**FIG.** 5 illustrates a flowchart of a method 500 for stent delivery and positioning for transluminal application in accordance with aspects of the present disclosure.

At 505, the method may include positioning the stent in an undeployed configuration through an access site in a wall of a first body lumen. The operations of 505 may be performed in accordance with examples as disclosed herein.

At 510, the method may include retracting an outer sheath proximally and past an anchoring component disposed at a distal portion of an inner tubular member based at least in part on positioning the stent, wherein a distal portion of the stent is disposed coaxially along the anchoring component such that the distal portion of the stent is disposed between the anchoring component and the outer sheath while the stent is in the undeployed configuration. The operations of 510 may be performed in accordance with examples as disclosed herein.

At 515, the method may include retaining the distal portion of the stent in place along the inner tubular member as the outer sheath is retracted past the anchoring component based at least in part on the distal portion of the stent being disposed between the anchoring component and the outer sheath. The operations of 515 may be performed in accordance with examples as disclosed herein.

At 520, the method may include deploying the distal portion of the stent from the outer sheath into a deployed configuration within the first body lumen based at least in part on retracting the outer sheath past the anchoring component. The operations of 520 may be performed in accordance with examples as disclosed herein.

At 525, the method may include expanding a proximal portion of the stent from within the outer sheath such that upon fully exiting the outer sheath, the proximal portion expands to a deployed configuration within a second body lumen. The operations of 525 may be performed in accordance with examples as disclosed herein.

It should be noted that these methods describe possible implementation, and that the operations and the steps may be rearranged or otherwise modified such that other implementations are possible. In some examples, aspects from two or more of the methods may be combined. For example, aspects of each of the methods may include steps or aspects of the other methods, or other steps or techniques described herein.

The description herein is provided to enable a person skilled in the art to make or use the disclosure. Various modifications to the disclosure will be readily apparent to those skilled in the art, and the generic principles defined herein may be applied to other variations without departing from the scope of the disclosure. Thus, the disclosure is not to be limited to the examples and designs described herein but is to be accorded the broadest scope consistent with the principles and novel features disclosed herein.

While several embodiments of the present disclosure have been described and illustrated herein, those of ordinary skill in the art will readily envision a variety of other means or structures for performing the functions or obtaining the results or one or more of the advantages described herein, and each of such variations or modifications is deemed to be within the scope of the present disclosure. More generally, those skilled in the art will readily appreciate that all parameters, dimensions, materials, and configurations described herein are meant to be exemplary and that the actual parameters, dimensions, materials, or configurations will depend upon the specific application or applications for which the teachings of the present disclosure is/are used.

Those skilled in the art will recognize or be able to ascertain using no more than routine experimentation, many equivalents to the specific embodiments of the disclosure described herein. It is, therefore, to be understood that the foregoing embodiments are presented by way of example only and that, within the scope of the appended claims and equivalents thereto, the disclosure may be practiced otherwise than as specifically described and claimed. The present disclosure is directed to each individual feature, system, article, material, kit, or method described herein. In addition, any combination of two or more such features, systems, articles, materials, kits, or methods, if such features, systems, articles, materials, kits, or methods are not mutually inconsistent, is included within the scope of the present disclosure.

All definitions, as defined and used herein, should be understood to control over dictionary definitions, definitions in documents incorporated by reference, or ordinary meanings of the defined terms.

The indefinite articles "a" and "an," as used herein in the specification and in the claims, unless clearly indicated to the contrary, should be understood to mean "at least one." Also, as used herein, including in the claims, "or" as used in a list of items (for example, a list of items prefaced by a phrase such as "at least one of' or "one or more") indicates an inclusive list such that, for example, a list of at least one of A, B, or C means A or B or C or AB or AC or BC or ABC (i.e., A and B and C).

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The invention may be described by reference to the following numbered paragraphs:-
1. A method for delivering a stent, comprising:
   positioning the stent in an undeployed configuration through an access site in a wall of a first body lumen;
   retracting an outer sheath proximally and past an anchoring component disposed at a distal portion of an inner tubular member based at least in part on positioning the stent, wherein a distal portion of the stent is disposed coaxially along the anchoring component such that the distal portion of the stent is disposed between the anchoring component and the outer sheath while the stent is in the undeployed configuration;
   deploying the distal portion of the stent from the outer sheath into a deployed configuration within the first body lumen based at least in part on retracting the outer sheath past the anchoring component; and
   expanding a proximal portion of the stent from within the outer sheath such that upon fully exiting the outer sheath, the proximal portion expands to a deployed configuration within a second body lumen.
2. The method of paragraph 1, further comprising:
   retaining the distal portion of the stent in place along the inner tubular member as the outer sheath is retracted past the anchoring component based at least in part on the distal portion of the stent being disposed between the anchoring component and the outer sheath.
3. The method of paragraph 1, wherein deploying the distal portion of the stent further comprises:
   releasing the distal portion of the stent from the anchoring component and into the deployed configuration based at least in part on retracting the outer sheath past the anchoring component.
4. The method of paragraph 1, wherein deploying the distal portion of the stent further comprises:
   expanding the distal portion of the stent from the undeployed configuration to the deployed configuration while the outer sheath is retracted based at least in part on a pressure being released between the anchoring component and the outer sheath as the outer sheath is retracted.
5. The method of paragraph 1, further comprising:
   compressing the distal portion of the stent in the undeployed configuration between the anchoring component and the outer sheath, wherein deploying the distal portion of the stent is based at least in part on compressing the distal portion of the stent.
6. The method of paragraph 1, wherein deploying the distal portion of the stent further comprises:
   expanding a flared portion of the stent within the first body lumen based at least in part on retracting the outer sheath.
7. The method of paragraph 6, further comprising:
   anchoring the flared portion of the stent within the first body lumen such that the distal portion of the stent remains in a fixed position.
8. The method of paragraph 1, further comprising:
   compressing the distal portion of the stent against the wall of the first body lumen based at least in part on deploying the distal portion of the stent from the outer sheath.
9. The method of paragraph 1, further comprising:
   maintaining the anchoring component and the inner tubular member in a stationary position while retracting the outer sheath.
10. The method of paragraph 1, further comprising:
   expanding an entire portion of the stent such that at least a portion of the stent extends through the first body lumen and into the second body lumen.
11. The method of paragraph 1, further comprising:
   pulling the inner tubular member from a proximal end of the inner tubular member; and
   removing the anchoring component and the inner tubular member from the first body lumen and the second body lumen after the stent fully deploys.
12. The method of paragraph 1, wherein the inner tubular member comprises a proximal marker around the inner tubular member and positioned such that a proximal end of the stent abuts against the proximal marker while the stent is in the undeployed configuration, and wherein the method further comprises:
   aligning a distal end of the proximal marker with a wall of the second body lumen;
   maintaining the proximal marker in a stationary position while retracting the outer sheath based at least in part on aligning the distal end of the proximal marker; and
   withdrawing the outer sheath past the distal end of the proximal marker based at least in part on maintaining the proximal marker in the stationary position, wherein expanding the proximal portion of the stent is based at least in part on withdrawing the outer sheath past the proximal marker.
13. The method of paragraph 1, further comprising:
   withdrawing the outer sheath until the proximal portion of the stent fully exits the outer sheath based at least in part on deploying the distal portion of the stent.
14. The method of paragraph 1, further comprising:
   advancing a stent delivery apparatus through the first body lumen and the second body lumen before delivering the stent; and
   removing the stent delivery apparatus through the first body lumen and the second body lumen after expanding the stent.
15. The method of paragraph 14, wherein the stent delivery apparatus comprises an electrocautery tip coupled with a distal end of the inner tubular member, and wherein the method further comprises:
   applying energy, via the electrocautery tip, to the wall of the first body lumen; and
   accessing, via the access site, the first body lumen based at least in part on applying the energy to the wall of the first body lumen, wherein positioning the stent is based at least in part on accessing the first body lumen.
16. The method of paragraph 14, wherein the stent delivery apparatus comprises an isolation sheath, wherein the isolation sheath is disposed around the outer sheath, and wherein retracting the outer sheath further comprises:
   maintaining the isolation sheath in a stationary position while retracting the outer sheath; and
   withdrawing the outer sheath into the isolation sheath based at least in part on positioning the stent.
17. The method of paragraph 14, wherein the stent delivery apparatus comprises a guidewire, wherein the guidewire is slidably disposed within the inner tubular member, and wherein the method further comprises:
   advancing the guidewire through the access site before positioning the stent.

## Claims

1. A stent delivery system, comprising:
An outer sheath which positions the stent in an undeployed configuration through an access site in a wall of a first body lumen;
an anchoring component disposed at a distal portion of an inner tubular member, based at least in part on positioning the stent, past which the outer sheath may be retracted proximally, wherein a distal portion of the stent is disposed coaxially along the anchoring component such that the distal portion of the stent is disposed between the anchoring component and the outer sheath while the stent is in the undeployed configuration;
wherein the distal portion of the stent can be deployed from the outer sheath into a deployed configuration within the first body lumen based at least in part on retracting the outer sheath past the anchoring component; and
a proximal portion of the stent can be expanded from within the outer sheath such that upon fully exiting the outer sheath, the proximal portion expands to a deployed configuration within a second body lumen.

2. The stent delivery system according to claim 1, further comprising:
retaining the distal portion of the stent in place along the inner tubular member as the outer sheath is retracted past the anchoring component based at least in part on the distal portion of the stent being disposed between the anchoring component and the outer sheath.

3. The stent delivery system according to claim 1 or claim 2, wherein deploying the distal portion of the stent further comprises:
releasing the distal portion of the stent from the anchoring component and into the deployed configuration based at least in part on retracting the outer sheath past the anchoring component.

4. The stent delivery system according to any preceding claim, wherein deploying the distal portion of the stent further comprises:
expanding the distal portion of the stent from the undeployed configuration to the deployed configuration while the outer sheath is retracted based at least in part on a pressure being released between the anchoring component and the outer sheath as the outer sheath is retracted.

5. The stent delivery system according to any preceding claim, further comprising:
compressing the distal portion of the stent in the undeployed configuration between the anchoring component and the outer sheath, wherein deploying the distal portion of the stent is based at least in part on compressing the distal portion of the stent.

6. The stent delivery system according to any preceding claim, wherein deploying the distal portion of the stent further comprises:
expanding a flared portion of the stent within the first body lumen based at least in part on retracting the outer sheath, preferably
further comprising:
anchoring the flared portion of the stent within the first body lumen such that the distal portion of the stent remains in a fixed position.

7. The stent delivery system according to any preceding claim, further comprising:
compressing the distal portion of the stent against the wall of the first body lumen based at least in part on deploying the distal portion of the stent from the outer sheath.

8. The stent delivery system of any preceding claim, further comprising:
maintaining the anchoring component and the inner tubular member in a stationary position while retracting the outer sheath.

9. The stent delivery system of any preceding claim, further comprising:
expanding an entire portion of the stent such that at least a portion of the stent extends through the first body lumen and into the second body lumen.

10. The stent delivery system of any preceding claim, further comprising:
pulling the inner tubular member from a proximal end of the inner tubular member; and
removing the anchoring component and the inner tubular member from the first body lumen and the second body lumen after the stent fully deploys.

11. The stent delivery system of any preceding claim, wherein the inner tubular member comprises a proximal marker around the inner tubular member and positioned such that a proximal end of the stent abuts against the proximal marker while the stent is in the undeployed configuration, and wherein the method further comprises:
aligning a distal end of the proximal marker with a wall of the second body lumen;
maintaining the proximal marker in a stationary position while retracting the outer sheath based at least in part on aligning the distal end of the proximal marker; and
withdrawing the outer sheath past the distal end of the proximal marker based at least in part on maintaining the proximal marker in the stationary position, wherein expanding the proximal portion of the stent is based at least in part on withdrawing the outer sheath past the proximal marker.

12. The stent delivery system of any preceding claim, further comprising:
withdrawing the outer sheath until the proximal portion of the stent fully exits the outer sheath based at least in part on deploying the distal portion of the stent.

13. The stent delivery system of any preceding claim, further comprising:
advancing a stent delivery apparatus through the first body lumen and the second body lumen before delivering the stent; and
removing the stent delivery apparatus through the first body lumen and the second body lumen after expanding the stent; preferably
wherein the stent delivery apparatus comprises an electrocautery tip coupled with a distal end of the inner tubular member, and wherein the method further comprises:
applying energy, via the electrocautery tip, to the wall of the first body lumen; and
accessing, via the access site, the first body lumen based at least in part on applying the energy to the wall of the first body lumen, wherein positioning the stent is based at least in part on accessing the first body lumen.

14. The stent delivery system of claim 13, wherein the stent delivery apparatus comprises an isolation sheath, wherein the isolation sheath is disposed around the outer sheath, and wherein retracting the outer sheath further comprises:
maintaining the isolation sheath in a stationary position while retracting the outer sheath; and
withdrawing the outer sheath into the isolation sheath based at least in part on positioning the stent.

15. The stent delivery system of claim 13 or claim 14, wherein the stent delivery apparatus comprises a guidewire, wherein the guidewire is slidably disposed within the inner tubular member, and wherein the method further comprises:
advancing the guidewire through the access site before positioning the stent.
